# EUROPEAN PATENT APPLICATION

(11) **EP 1 669 604 A2**
(43) Date of publication of application: **14.06.2006**
(21) Application number: 05257501.6
(22) Date of filing: 06.12.2005
(51) Int. Cl.: F04B 53/16, F04B 53/00

(54) **Reusable pump cartridge**

(30) Priority: 07.12.2004 US 904959
(71) Applicant: Depuy Mitek, Inc., Norwood, MA 02062 (US)
(72) Inventor: Sengun, Mehmet Z., Framingham, MA 01702 (US); Weinert, Christopher G., Mansfield, MA 02048 (US); Ranucci, Kevin J., Warwick, RI 02886 (US); McRury, Ian D., Medway, MA 02053 (US)
(74) Representative: Fisher, Adrian John

(57) **Abstract**

A pump cartridge for use in a high pressure fluid jet system is provided. In an exemplary embodiment, the pump cartridge can be adapted to couple to a drive mechanism for driving fluid from a fluid source, through the pump cartridge, to a fluid jet delivery device, and the pump cartridge can be reusable to allow the pump cartridge to be sterilized between uses.

## Description

### FIELD OF THE INVENTION

The present invention relates to high pressure fluid jet tools, and in particular to a reusable pump cartridge for use with a high pressure fluid jet.

### BACKGROUND OF THE INVENTION

High pressure fluid jets for cutting tissue can offer several advantages over traditional cutting tools. In particular, high pressure fluid jets tend to emulsify soft tissue, thus avoiding thermal damage and necrosis which can arise from using laser cutters and electrosurgical cutters. The emulsified tissue can also be easily transported by aspiration away from the surgical site. Indeed, the fact that many fluid jet cutting devices include aspiration and evacuation as an integral portion of the device can be an added benefit for many surgical procedures.

One drawback with current high pressure fluid jets used in surgical procedures is that they require a sterile fluid flow pathway from an external pump mechanism to a nozzle that forms the fluid jet. Most current high pressure fluid jets use a disposable fluid pathway that is sterilized during manufacturing, and that is discarded after use. The materials and the configuration of the fluid pathway often prevent the device from being sterilized after use.

Accordingly, there remains a need for an improved high pressure fluid jet, and in particular for a reusable pump cartridge for a high pressure fluid jet system.

### SUMMARY OF THE INVENTION

In one exemplary embodiment, a reusable pump cartridge is provided for use in a high pressure fluid jet system. The pump cartridge can be adapted to couple to a drive mechanism for driving fluid through the pump cartridge. Between uses, the pump cartridge can be disengaged from the drive mechanism, sterilized, and reused.

While the pump cartridge can have a variety of configurations, in one exemplary embodiment the pump cartridge can include a housing having a chamber adapted for fluid flow therethrough, and a drive assembly disposed therein and adapted to drive fluid through the chamber when the housing is coupled to a drive mechanism. The chamber can be adapted to have a sealed configuration when the housing is coupled to the drive mechanism, and the chamber can be adapted to have an open configuration when the housing is disengaged from a drive mechanism to allow the pump cartridge to be sterilized. In one exemplary embodiment, the chamber can include a one-way inlet valve that is adapted to allow fluid to flow into the chamber, and a one-way outlet valve that is adapted to allow fluid to flow out of the chamber. By way of non-limiting example, the inlet and outlet valves can be ball-in-aperture valves.

The drive assembly can have a variety of configurations, but in one exemplary embodiment the drive assembly can include a piston that is movably disposed through a seal that separates the chamber from the drive assembly. The piston can be adapted to receive a force from a push rod on a drive mechanism for moving the piston relative to the seal to drive fluid through the chamber. In one exemplary embodiment, the piston can float within the housing such that it is movable about a longitudinal axis thereof. The drive assembly can also include a biasing element that is coupled to the piston and that is effective to bias the piston to a first position in which the piston is fully removed from the chamber and the seal is open.

In another aspect of the present invention, a reusable pump cartridge is provided and it can include a housing that is removably matable to a drive mechanism and that includes a fluid flow chamber, a coupling chamber, and a seal separating the fluid flow chamber and the coupling chamber. The reusable pump cartridge can also include a piston that is movably disposed through the seal and that includes a receiving portion disposed within the coupling chamber and that is adapted to receive a push rod on a drive mechanism for moving the piston relative to the seal to drive fluid through the fluid flow chamber. The pump cartridge can also include a biasing element coupled to the piston and effective to bias the piston to a first position in which the piston is fully disposed within the pump chamber and the seal is open. The biasing element can also have a variety of configurations, but in one embodiment the biasing element can be a coil spring that is disposed around at least a portion of the piston to apply a biasing force to the second end of the head of the piston.

The piston can have a variety of configurations, and in one exemplary embodiment the piston can be, for example, an elongate shaft having an enlarged head formed thereon. The head can include a substantially planar surface for receiving a push rod, or in another embodiment it can include a recess formed therein and defining the receiving portion. In another exemplary embodiment, the piston can be formed from an autoclavable material, such as sapphire. The seal in the housing can also be formed from an autoclavable material, such as, for example, an impregnated fluoropolymer, such as a polymer-filled TEFLON®.

Exemplary methods for using a pump cartridge are also provided.

### BRIEF DESCRIPTION OF THE DRAWINGS

The invention will be more fully understood from the following detailed description taken in conjunction with the accompanying drawings, in which:
FIG. 1 is an illustration of a high pressure fluid jet system having a pump console with a pump cartridge connected thereto and in communication with a fluid jet delivery device in accordance with one exemplary embodiment of the present invention;
FIG. 2 is a perspective view of the pump cartridge of the high pressure fluid jet system of FIG. 1;
FIG. 3A is a cross-sectional view of the pump cartridge shown in FIG. 2;
FIG. 3B is a perspective view of a portion of the pump cartridge shown in FIG. 3A;
FIG. 4A is a cross-sectional view of another exemplary embodiment of a pump cartridge for use with a high pressure fluid jet system;
FIG. 4B is a cross-sectional view of a portion of the pump cartridge shown in FIG. 4A; and
FIG. 5 is a cross-sectional view of one exemplary embodiment of a valve mechanism for use with a pump cartridge.

### DETAILED DESCRIPTION OF THE INVENTION

The present invention provides a pump cartridge for use in a high pressure fluid jet system. The pump cartridge can be adapted to couple to a drive mechanism for driving fluid from a fluid source, through the pump cartridge, to a fluid jet delivery device. In an exemplary embodiment, the pump cartridge can be reusable to allow the pump cartridge to be sterilized between uses. A person skilled in the art will appreciate that the pump cartridge can be used with a variety of high pressure fluid jet systems for use in a variety of applications.

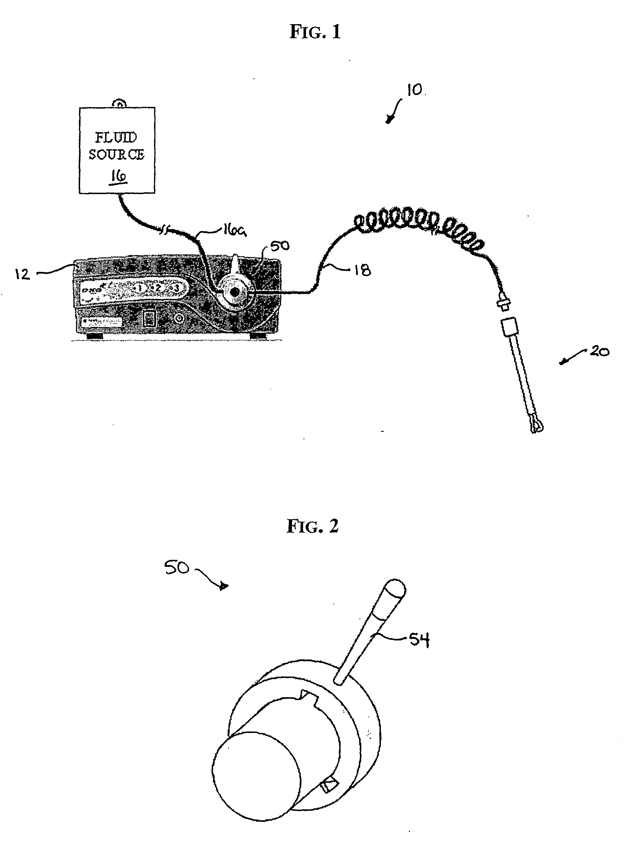

FIG. 1 illustrates one exemplary embodiment of a high pressure fluid jet system 10. As shown, the system 10 can include a drive mechanism 12 and a reusable pump cartridge 50 that can be releasably attached to the drive mechanism 12. The system 10 can also include a fluid source 16, such as a saline bag, for delivering fluid to the reusable pump cartridge 50. The fluid source 16 can be coupled to the pump cartridge 50 using a variety of techniques, but in one exemplary embodiment the fluid source 16 includes a tube 16a that extends between the fluid source 16 and the pump cartridge 50. The system 10 can also include a fluid jet delivery device, which in the illustrated embodiment includes a fluid delivery tube 18 and fluid jet device 20, for receiving fluid from the pump cartridge 50 and forming a high pressure fluid jet. While the high pressure fluid jet can operate at various pressures depending on the intended use, in one exemplary embodiment the high pressure fluid jet system 10 is adapted to operate at a pressure in the range of about 1,000 to 20,000 psi, and more preferably in the range of about 5,000 to 15,000 psi. A person skilled in the art will appreciate that the high pressure fluid jet system can include a variety of other components, and that each component can have a variety of configurations. Moreover, the components can be integrally formed with one another or they can be removably attached to one another. A person skilled in the art will also appreciate that the exemplary pump cartridges disclosed herein can be used with a variety of other fluid jet systems, and that the exemplary fluid jet system disclosed herein is merely disclosed for reference purposes.

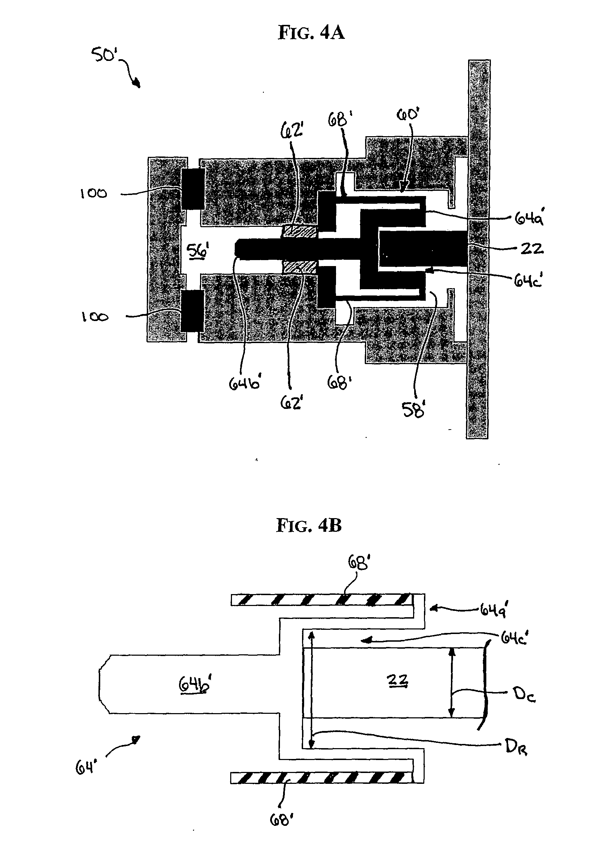

While virtually any drive mechanism known in the art can be used, in one exemplary embodiment the drive mechanism can be part of a pump console 12 for pumping fluid through the pump cartridge 50 at a controlled rate. The exemplary pump console 12 can include a push rod 22 (shown in FIG. 4) that is driven by a motor disposed within the pump console 12, and controls for allowing a user to input the desired pump parameters. The push rod 22 can have a variety of shapes and sizes, but in one exemplary embodiment the push rod 22 has a shape and size that enables it to extend from the pump console 12 and into the pump cartridge 50 to apply a force to a piston disposed within the pump cartridge 50, as will be discussed in more detail below. In use, the motor (not shown) is effective to reciprocate the push rod 22 along its axis, thereby reciprocating the piston disposed within the pump cartridge 50 to pump fluid through the cartridge 50, as will also be discussed in more detail below.

The fluid delivery tube 18 can also have a variety of configurations. In one exemplary embodiment, the fluid delivery tube 18 can be formed from a material which has sufficient burst strength to safely deliver fluid at a high pressure to the fluid jet device 20, and which has good maneuverability for the surgeon. As shown in FIG. 1A, the fluid delivery tube 18 can be coiled to provide good maneuverability. The fluid delivery tube 18 can also include connectors, which in an exemplary embodiment can be hand tightened, to connect the ends of the fluid delivery tube 18 to the pump cartridge 50 and the fluid jet device 20, where detachable components are desired. As previously indicated, the fluid delivery tube 18 can be integrally formed with or fixedly mated to the pump cartridge 50 and/or the fluid jet device 20.

The fluid jet device 20 can also have a variety of configurations, and virtually any device for forming a high pressure fluid jet can be used with the various embodiments disclosed herein. In the illustrated exemplary embodiment, the fluid jet device 20 is in the form of a hand-held wand that includes a lumen in communication with the delivery tube 18 and a nozzle for forming a high pressure fluid jet. The fluid jet device 20 can also include an evacuation lumen for collecting the fluid jet, as well as a variety of other features for facilitating use of the device. By way of non-limiting example, one exemplary embodiment of a fluid jet device is disclosed in commonly owned U.S. Patent Appl. No. 10/904,456 filed on November 11, 2004 and entitled "Methods and Devices for Selective Bulk Removal and Precision Sculpting of Tissue" by McRury et al.

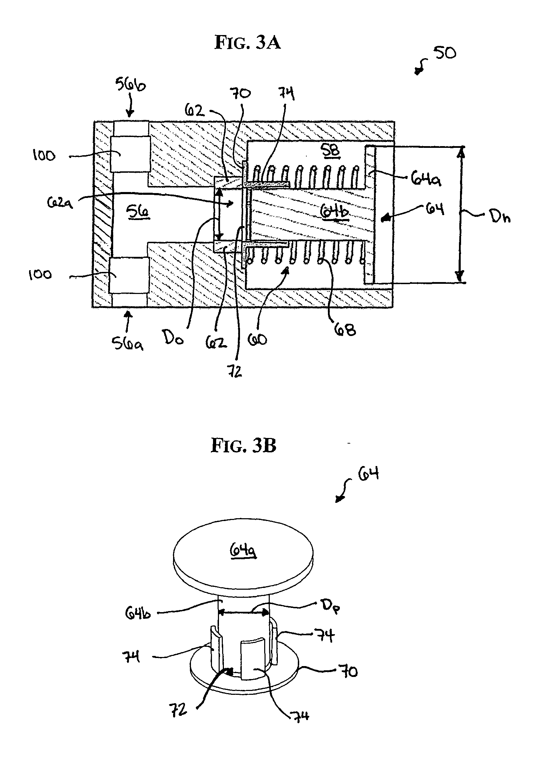

As previously indicated, the high pressure fluid jet system 10 can include a pump cartridge 50 that is adapted to couple to the pump console 12, and that is disposed between and effective to transfer fluid from the fluid source 16 to the fluid jet delivery device 18, 20. The pump cartridge 50 can have a variety of configurations, but in one exemplary embodiment, shown in FIGS. 3A and 3B, the pump cartridge 50 can be in the form of a housing having a first or fluid flow chamber 56 that is adapted to deliver fluid from the fluid source 16 to the fluid delivery tube 18 and pressure jet device 20, and a second or coupling chamber 58 having a drive assembly 60 disposed therein and adapted to drive fluid through the fluid flow chamber 56 when the cartridge 50 is coupled to the console 12. The exemplary pump cartridge 50 can also be adapted to be sterilized, thus allowing the pump cartridge to be reused. This can be achieved by configuring the pump cartridge 50 such that the internal chambers, e.g., the fluid flow chamber 56 and the coupling chamber 58, of the cartridge 50 are in an open configuration when the pump cartridge 50 is disengaged from the console 12.

A person skilled in the art will appreciate that the pump cartridge is described as having a second or coupling "chamber" for reference purposes only, and that the components disposed within the second chamber of the pump cartridge does not necessarily need to be disposed within a "chamber" or a defined space.

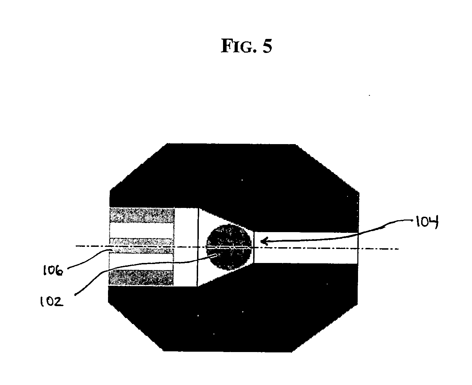

The fluid flow chamber 56 can have a variety of configurations, but in an exemplary embodiment, as shown, it includes an inlet port 56a that is adapted to mate to the fluid source 16, and an outlet port 56b that is adapted to mate to the fluid delivery tube 18. The inlet and outlet ports 56a, 56b can each include a valve mechanism disposed therein for controlling fluid flow therethrough. While a variety of valve mechanisms can be used, in one exemplary embodiment the inlet and outlet ports 56a, 56b each include a one-way ball-in-aperture valve 100 having a ball 102 that is sits within an aperture 104, as shown in more detail in FIG. 5. The valve 100 can also include a ball-retaining member 106, such as a rod extending across the inlet and/or outlet ports 56a, 56b, that is adapted to retain the ball 102 within the valve housing 100. In use, when the pump cartridge 50 is attached to the console 12, the ball 102 can prevent fluid from flowing out the inlet 56a and in the outlet 56b in the fluid flow chamber 56. When the pump cartridge 50 is disengaged from the console 12, the cartridge 50 can be laid on its side such that the ball 102 moves away from the aperture 104, thereby allowing the pump cartridge 50 to be sterilized as the fluid flow chamber 56 is in an open configuration, as will be discussed in more detail below.

As is further shown in FIGS. 3A and 3B, the exemplary fluid flow chamber 56 can be in communication with the coupling chamber 58 to allow the drive assembly 60 within the coupling chamber 58 to drive fluid through the fluid flow chamber 56. While various techniques can be used to couple the two chambers 56, 58, in the illustrated exemplary embodiment a seal 62 can be disposed between the two chambers 56, 58. The seal 62 can have a variety of shapes and sizes, but in one exemplary embodiment the seal 62 separates the fluid flow chamber 56 and the coupling chamber 58. The seal 62 can, however, include an opening 62a formed therein for slidably receiving a piston 64 that is part of the drive assembly 60 disposed within the coupling chamber 58. As will be discussed in more detail below, the piston 64 can be adapted to extend through the opening 62a in the seal 62 when the pump cartridge 50 is attached to the console 12, and it can retract into the coupling chamber 58 when the pump cartridge 50 is disengaged from the pump console 12 such that the fluid flow chamber 56 and the coupling chamber 58 are in communication with one another, thereby allowing the pump cartridge 50 to have an open configuration for sterilization. Thus, while the opening 62a in the seal 62 can have virtually any shape and size, in an exemplary embodiment the opening 62a has a shape and size that complements a shape and size of the piston. For example, the opening can be circular to match a cylindrical-shaped piston. A person skilled in the art will appreciate that the seal 62 can be formed from a variety of materials. Exemplary materials include, by way of non-limiting example, an impregnated fluoropolymer, such as a polymer-filled TEFLON®.

The coupling chamber 58 of the pump cartridge 50 can also have a variety of configurations, but as indicated above an exemplary coupling chamber 58 includes a drive assembly 60 disposed therein that is adapted to receive a force from the push rod 22 on the console 12, and that is adapted to drive fluid through the fluid flow chamber 56. As shown in FIGS. 3A and 3B, an exemplary drive assembly 60 can include a piston 64 having a first portion or a head 64a that is adapted to receive a force applied by the push rod 22 on the console 12, and a second portion 64b that extends through the seal 62 and into the fluid flow chamber 56 for driving fluid therethrough. The first and second portions 64a, 64b can have virtually any shape and size, but in one exemplary embodiment the second portion 64b of the piston 64 can have a generally elongate cylindrical shape with an extent, e.g., a diameter *D*_{*p*} that substantially corresponds to an extent, e.g., a diameter *D*_{*o*}, of the opening 62a in the seal 62, and the first portion or head 64a of the piston 64 can have a substantially planar configuration to allow the push rod 22 to abut there against. The head 64a can be enlarged such that it has an extent, e.g., a diameter *D*_{*h*}, that is greater than the diameter *D*_{*p*} of the second portion 64b, and that is greater than an extent, e.g., a diameter *D*_{*c*}, of the push rod 22 (shown in FIGS. 4A and 4B). Such a configuration allows the push rod 22 to contact any portion of the piston 64, i.e., it allows a central axis of the push rod 22 to be aligned or misaligned with a central axis of the piston 64. Thus, a mechanical interlocking fixation between the push rod 22 and the piston 64 is not necessary as the components do not need to be axially aligned. Accordingly, the piston 64 can float within the pump cartridge 50 and relative to the push rod 22. As a result, the push rod 22 will not cause the piston 64 to be misaligned with the seal 62, thereby reducing or avoiding potential wear on the seal 62. While not shown, the head 64a of the piston 64 can include one or more openings or other features formed therein to facilitate sterilization of the pump cartridge 50, and in particular to prevent the piston 64 from sealing the second chamber 58 when the pump cartridge is disengaged from the drive mechanism 12.

In another exemplary embodiment, the piston 64 can include a recess formed in a proximal end thereof for receiving the push rod 22. FIGS. 4A and 4B illustrate another embodiment of a pump cartridge 50' that is similar to pump cartridge 50 shown in FIGS. 3A and 3B, but that includes a recess 64c' formed in the piston 64' for receiving the push rod 22. In particular, the piston 64' can have an enlarged head 64a' formed thereon with a recess 64c' formed within the head 64'. The recess 64c' can have virtually any shape and size, but in an exemplary embodiment it has an extent, e.g., a diameter *D*_{*R*}, that is substantially larger than an extent, e.g., the diameter *D*_{*c*}, of the push rod 22, as shown in FIG. 4B. Again, such a configuration allows the push rod 22 to be received within any portion of the recess 64c', and does not require a mechanical connection to axially align the piston 64 with the push rod 22. Accordingly, the piston 64' can float within the pump cartridge 50 and relative to the push rod 22. As a result, the push rod 22 will not cause the piston 64' to be misalignment with the seal 62, thereby reducing or avoiding potential wear on the seal 62'.

The piston 64, 64' can also be formed from a variety of materials, but in one exemplary embodiment the piston 64, 64' is formed from a material that is impervious to sterilization. One suitable exemplary material is sapphire. Sapphire is also particularly advantageous in that the use of a sapphire piston can be highly polished and is extremely hard such that it will remain stable in high temperature and high pressure environments.

Referring back to FIGS. 3A and 3B, while the piston 64 can float within the coupling chamber 58 and it does not need to be axially aligned with or mated to the push rod 22 on the console 12, the pump cartridge 50 can include features to facilitate axial alignment of the piston 64 with the opening 62a in the seal 62. Various techniques can be used to achieve this, but in one exemplary embodiment the pump cartridge 50 can include an alignment mechanism, such as a ring member 70 shown in FIGS. 3A and 3B, that is adapted to facilitate alignment of the second elongate portion 64b of the piston 64 with the seal 62. The exemplary ring member 70 can have a substantially cylindrical shape with an opening 72 formed therein for slidably receiving the elongate portion 64b of the piston 64. It can also include one or more projections 74 for guiding the piston 64 into the opening 72 during initial engagement, and/or for facilitating sterilization of the device as the projections will allow steam communication between the chambers. For example, as shown in FIG. 3B, the ring member 70 is castellated. FIG. 3A illustrates the ring member 70 disposed within the pump cartridge 50, and as shown the ring member 70 can be fixedly attached to the housing within the coupling chamber 58 such that the opening 72 is aligned with the opening 62a of the seal 62. As a result, when the piston 64 moves toward the seal 62, the projections 74 guide the piston 64 toward the opening 72, thereby substantially aligning the piston 64 with the opening 62a in the seal 62. The ring member 70 can also be effective to maintain the seal 62 in a substantially fixed position within the pump cartridge 50.

In another embodiment, shown in FIGS. 4A and 4B, the piston 64' can be self-aligning. In particular, a biasing element 68', which will be discussed in more detail below, can be disposed around the piston 64 and used to maintain and substantially align the piston 64 with the opening in the seal 62a. The piston 64, however, can move laterally about its axis such that the piston 64 can self-align with the seal opening 62a. A person skilled in the art will appreciate that a variety of other techniques can be used to axially align the piston 64 with the seal 62.

As indicated above, the drive assembly 60, 60' in the pump cartridge 50, 50' can also include a biasing element 68 that is adapted to bias the piston 64, 64' into the coupling chamber 58, 58'. While virtually any biasing element 68 can be used, in the exemplary embodiment shown in FIG. 3A the biasing element 68 can be in the form of a coil spring that is disposed around the elongate portion 64b, 64b' of the piston 64, 64' and that is positioned between the head 64a of the piston 64 and the ring member 70. FIGS. 4A and 4B also illustrate a coil spring 68'. As noted above, the spring 68' can maintain and substantially align the piston 64' with the seal 62 while allowing some lateral movement of the piston 64' such that the piston 64' is self-aligning with the seal 62.

In use, when the pump cartridge 50 is attached to the console 12, the biasing element 68 will apply a force in the direction of the console 12, thereby forcing the piston 64 into contact with the push rod 22. As a result, when the motor is actuated and the push rod 22 is reciprocated along its axis, it will apply a counter-force to the biasing element 68, thereby reciprocating the piston 64 through the seal 62 and into the fluid flow chamber 56 to pump fluid therethrough. When the pump cartridge 50 is removed or disengaged from the console 12, the biasing element 68 can force the piston 64 out of the opening 62a in the seal 62 and completely into the second chamber 58 such that the fluid flow chamber 56 and the coupling chamber 58 are open or in communication with one another. The pump cartridge 50 can also be turned on its side to cause the valves 100 to open, such that the entire pump cartridge 50 is open. As a result, the pump cartridge 50 can be sterilized, e.g., using an autoclave, cleaning agents, steam, gas, etc., for reuse.

A person skilled in the art will appreciate that the cartridge 50 can be coupled to the console 12 using a variety of engagement techniques. By way of non-limiting example, suitable engagement mechanisms include a twist-lock mechanism, a threaded mechanism, or any other technique known in the art. The pump cartridge 50 can also include a lever 54, shown in FIG. 2, for facilitating attachment to and removal from the pump console 12. For example, the lever 54 can be used to rotate the pump cartridge 50 relative to the console 12, thereby causing flanges, threads, or some other engagement mechanism on the cartridge 50 to engage the console 12. In certain exemplary embodiments, the pump cartridge 50 can releasably engage the console 12.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A reusable pump cartridge, comprising:
a housing having a chamber adapted to allow fluid flow therethrough, and a drive assembly adapted to drive fluid through the chamber when the housing is coupled to a drive mechanism, the chamber being adapted to have a sealed configuration when the housing is coupled to the drive mechanism, and the chamber being adapted to be in an open configuration when the housing is disengaged from a drive mechanism such that the housing, the chamber, and the drive assembly can be steam sterilized between uses.

2. The reusable pump cartridge of claim 1, wherein the chamber includes an inlet, and outlet, and an opening formed between the chamber and the drive assembly.

3. The reusable pump cartridge of claim 2, further comprising a seal disposed within the opening and adapted to have an open configuration when the housing is disengaged from a drive mechanism, and adapted to receive a portion of the drive assembly when the housing is coupled to the drive mechanism such that the chamber is sealed.

4. The reusable pump cartridge of claim 3, wherein the drive assembly includes a piston movably disposed through the seal, the piston being adapted to receive a push rod on a drive mechanism for moving the piston relative to the seal to drive fluid through the chamber.

5. The reusable pump cartridge of claim 4, wherein the piston floats within the housing such that it is movable about a longitudinal axis thereof.

6. The reusable pump cartridge of claim 4, further comprising a biasing element coupled to the piston and effective to bias the piston to a first position in which the piston is fully removed from the chamber and the seal is open.

7. The reusable pump cartridge of claim 2, wherein the inlet in the chamber includes a one-way inlet valve adapted to allow fluid to flow into the chamber, and the outlet in the chamber includes a one-way outlet valve adapted to allow fluid to flow out of the chamber.

8. The reusable pump cartridge of claim 7, wherein the inlet and outlet valves comprise ball-in-aperture valves.

9. A reusable pump cartridge, comprising:
a housing removably matable to a drive mechanism, the housing including a fluid flow chamber, a coupling chamber, and a seal separating the fluid flow chamber and the coupling chamber;
a piston movably disposed through the seal and including a receiving portion disposed within the coupling chamber and adapted to receive a push rod on a drive mechanism for moving the piston relative to the seal to drive fluid through the fluid flow chamber; and
a biasing element coupled to the piston and effective to bias the piston to a first position in which the piston is fully disposed within the pump chamber and the seal is open.

10. The reusable pump cartridge of claim 9, wherein the piston floats within the housing such that it is movable about a longitudinal axis thereof.

11. The reusable pump cartridge of claim 9, wherein the receiving portion comprises a substantially planar surface.

12. The reusable pump cartridge of claim 9, wherein the piston comprises an elongate shaft and the receiving portion comprises an enlarged head formed on an end of the elongate shaft, and wherein the enlarged head includes a substantially planar surface for receiving a push rod on a drive mechanism.

13. The reusable pump cartridge of claim 9, wherein the piston is formed from an autoclavable material.

14. The reusable pump cartridge of claim 9, wherein the piston is formed from sapphire.

15. The reusable pump cartridge of claim 9, wherein the seal is formed from an autoclavable material.

16. The reusable pump cartridge of claim 9, wherein the seal is formed from an impregnated fluoropolymer.

17. The reusable pump cartridge of claim 9, wherein the biasing element comprises a coil spring disposed around at least a portion of the piston.

18. The reusable pump cartridge of claim 9, wherein the piston comprises a head having a first end defining the receiving portion, and a second end having an elongate shaft extending therefrom and movably disposed through the seal.

19. The reusable pump cartridge of claim 18, wherein the biasing element extends around a portion of the elongate shaft and applies a biasing force to the second end of the head.

20. The reusable pump cartridge of claim 9, wherein the fluid flow chamber includes a one-way inlet valve adapted to allow fluid to flow into the fluid flow chamber, and a one-way outlet valve adapted to allow fluid to flow out of the fluid flow chamber.

21. The reusable pump cartridge of claim 20, wherein the inlet and outlet valves comprise ball-in-aperture valves.

22. A reusable pump cartridge, comprising:
a housing adapted to couple to a fluid source, a fluid delivery tool, and a drive mechanism for driving fluid from the fluid source, through the housing, to the fluid delivery tool;
wherein the housing is adapted to have a first, sealed configuration when the housing is coupled to a fluid source, a fluid delivery tool, and a drive mechanism, and a second, open configuration when the housing is disengaged from the fluid source, the fluid delivery device, and the drive mechanism; and
wherein the housing is adapted to be steam sterilized in the second, open configuration.

23. A method for using a pump cartridge, comprising:
coupling a drive assembly in a pump cartridge to a drive mechanism, and coupling a fluid flow chamber in the pump cartridge to a fluid source and a fluid delivery tool;
activating the drive mechanism to drive fluid from the fluid source, through the fluid flow chamber, to the fluid delivery tool;
disengaging the pump cartridge from the drive mechanism, the fluid source, and the fluid delivery tool;
sterilizing the pump cartridge; and
repeating the steps of coupling and activating.

24. The method of claim 23, wherein sterilizing the pump cartridge comprises autoclaving the pump cartridge.

25. The method of claim 23, wherein the fluid delivery tool comprises a high pressure fluid jet.
